# EUROPEAN PATENT APPLICATION

(11) **EP 4 501 373 A1**
(43) Date of publication of application: **05.02.2025**
(21) Application number: 23774594.8
(22) Date of filing: 10.03.2023
(51) Int. Cl.: A61M 5/142, A61M 5/145, A61M 5/168

(54) **CONTINUOUS INFUSION DEVICE AND MEDICAL SYSTEM**

(30) Priority: 25.03.2022 JP 2022049284
(71) Applicant: Terumo Kabushiki Kaisha, Tokyo 151-0072 (JP)
(72) Inventor: YOSHIKAWA, Hiroki, Ashigarakami-gun, Kanagawa 259-0151 (JP); HATA, Suguru, Ashigarakami-gun, Kanagawa 259-0151 (JP)
(74) Representative: Casalonga
(86) International application number: PCT/JP2023/009312
(87) International publication number: WO 2023/181995

(57) **Abstract**

A device includes: a main body portion (10A) having an inner cavity (11A); a plug (40A) that is slidable in a liquid-tight manner in the inner cavity (11A); a pressing portion (30A) that is disposed on the upstream side of the plug (40A) in the inner cavity (11A) and presses the plug (40A) toward the downstream side; a substance (X) to be administered that is accommodated on the downstream side of the plug (40A) in the inner cavity (11A) and is released into the living body by sliding of the plug (40A) toward the downstream side; and a release portion (50A) that is disposed on the distal end side of the main body portion (10A), and releases the substance (X) to be administered pushed out by the plug (40A) into the living body, the device being indwelled in a living body for sustainably extendedly releasing the substance (X) to be administered, and including: a notification unit (60A) that performs a notification operation for notifying abnormality to the outside when an internal pressure of the inner cavity (11A) exceeds a predetermined threshold value due to stop of movement of the plug (40A).

## Description

### Technical Field

The present invention relates to a sustained administration device that sustainably administers a substance to be administered such as a drug solution in a state where the device is indwelled in a living body, and a medical system provided with the device.

### Background Art

Patent Literature 1 discloses an osmotic delivery device that is a sustained administration device that extendedly releases, in a sustained manner for a long period of time, a drug that is a substance to be administered in a state where the device is embedded in a living body for a long period of time.

The device of Patent Literature 1 is roughly constituted with a hollow container (main body portion), a piston (plug) slidably held in the container, an osmotic engine (pressing portion) serving as a drive source for pressing the piston, a semipermeable membrane (liquid permeation portion) that is disposed on a proximal end side of the container and allows a liquid component in a body fluid (extracellular fluid) to permeate the osmotic engine, and a delivery orifice (release portion) that is disposed on a distal end side of the container and releases a drug pressed by the piston. The container includes a drug accommodating space (second space) defined by a piston and located on a distal end side of the container with the piston as a boundary, and an osmotic agent accommodating space (first space) located on a proximal end side of the container opposite to the second space with the piston as a boundary, and is configured such that the osmotic agent is swollen by a liquid component in a body fluid that has permeated through the semipermeable membrane using the principle of osmotic pressure, the piston is slid toward the distal end side along with an increase in internal pressure of the first space due to swelling of the osmotic agent, and the drug accommodated in the second space can be released into a living body.

### Citation List

### Patent Literature

Patent Literature 1: JP 2017-57212 A

### Summary of Invention

### Technical Problem

The device of Patent Literature 1 is a device that is indwelled in a living body and releases a substance to be administered extendedly for a certain period of time. Therefore, in a case where some kind of failure occurs in the device and the substance to be administered is not normally administered, there is a possibility that health damage of the patient is caused. In particular, a drug that exhibits a certain therapeutic effect in sustainable administration may cause serious health damage to a patient when a failure occurs in the device, and is therefore difficult to use. In addition, when the internal pressure of the device abnormally increases due to the stop of sliding of the plug, it may lead to excessive administration (burst) of the substance to be administered.

As described above, in the sustained administration device, it is important to immediately confirm from outside the living body whether or not the device function stops due to an abnormal increase in the internal pressure of the inner cavity or the like. However, with the conventional sustained administration device including the device of Patent Literature, there is no way to confirm the driving state from outside the living body after being indwelled in the living body.

At least one embodiment of the present invention has been made in view of the above circumstances, and specifically, it is an object of the present invention to provide a sustained administration device allowing confirmation of a driving state from outside a living body in a state of being indwelled in the living body, and a medical system provided with the device.

### Solution to Problem

A sustained administration device according to the present embodiment includes: a main body portion including an inner cavity; a plug that is slidable in a liquid-tight manner in the inner cavity; a pressing portion that is disposed on an upstream side of the plug in the inner cavity and presses the plug toward a downstream side; a substance to be administered that is accommodated on the downstream side of the plug in the inner cavity and is released into a living body by sliding of the plug toward the downstream side; and a release portion that is disposed on a distal end side of the main body portion and releases the substance to be administered pushed by the plug into the living body, the sustained administration device being indwelled in the living body to sustainably release the substance to be administered, in which the sustained administration device comprises a notification unit that notifies a stop state of the plug to the outside when an internal pressure of the inner cavity exceeds a predetermined threshold value due to stop of movement of the plug.

A medical system according to the present embodiment includes: the sustained administration device; and an external device that is disposed outside the living body and includes a detection unit that detects the notification operation of the notification unit.

### Advantageous Effects of Invention

According to at least one embodiment of the present invention, the driving state of the sustained administration device indwelled in the living body can be confirmed from outside the living body. Therefore, it is possible to avoid the risk of loss of the administration effect of the substance to be administered due to stop of extended release and the serious health hazard due to the burst of the substance to be administered. Therefore, even a drug that causes serious side effects when excessively administered can be used as a substance to be administered without concern, and the range of treatment options can be extended.

### Brief Description of Drawings

Fig. 1 is a functional diagram of a medical system according to a first embodiment.
Fig. 2 is a schematic cross-sectional view of the sustained administration device according to the first embodiment.
Fig. 3 is a partially enlarged cross-sectional view of the vicinity of the proximal end of the sustained administration device according to the first embodiment.
Fig. 4 is a functional diagram of a medical system according to a second embodiment.
Fig. 5 is a schematic cross-sectional view of the sustained administration device according to the second embodiment.
Fig. 6 is a partially enlarged cross-sectional view of the vicinity of the proximal end of the sustained administration device according to the second embodiment.
Fig. 7 is a functional diagram of a medical system according to a third embodiment.
Fig. 8 is a schematic cross-sectional view of the sustained administration device according to the third embodiment.
Fig. 9 is a partially enlarged cross-sectional view of the vicinity of the proximal end of the sustained administration device according to the third embodiment.
Fig. 10 is a partially enlarged cross-sectional view of a modification of the sustained administration device according to the third embodiment.

### Description of Embodiments

Hereinafter, a mode for carrying out the present invention will be described in detail with reference to the drawings. Embodiments herein are illustrated to embody the technical idea of the present invention and do not limit the present invention. Furthermore, other modes, examples, operation techniques, and the like, that could be conceived of by those skilled in the art without departing from the gist of the present invention are all included in the scope and gist of the present invention and included in the invention set forth in the claims and the scope of equivalents thereof.

Moreover, for convenience of illustration and ease of understanding, the drawings attached to the present specification may be schematically represented by changing a scale, an aspect ratio, a shape, and the like, from actual ones as appropriate, but are merely examples, and do not limit the interpretation of the present invention.

Note that, in the following description, ordinal numerals such as "first" and "second" will be given, but are used for convenience and do not define any order unless otherwise specified.

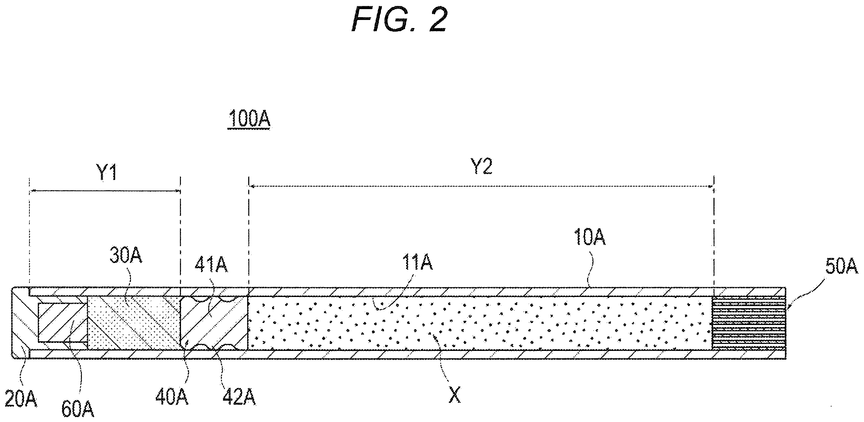

In the sustained administration device according to the first to third embodiments described below, the "distal end side (downstream side)" is a side on which the release portion is disposed and the substance X to be administered is administered, and the "proximal end side (upstream side)" is a side opposite to the distal end side and on which the liquid permeation portion is disposed and from which the liquid in the living body enters. For example, in Fig. 2, the distal end side is the right side in the drawing, and the proximal end side is the left side in the drawing.

### [First Embodiment]

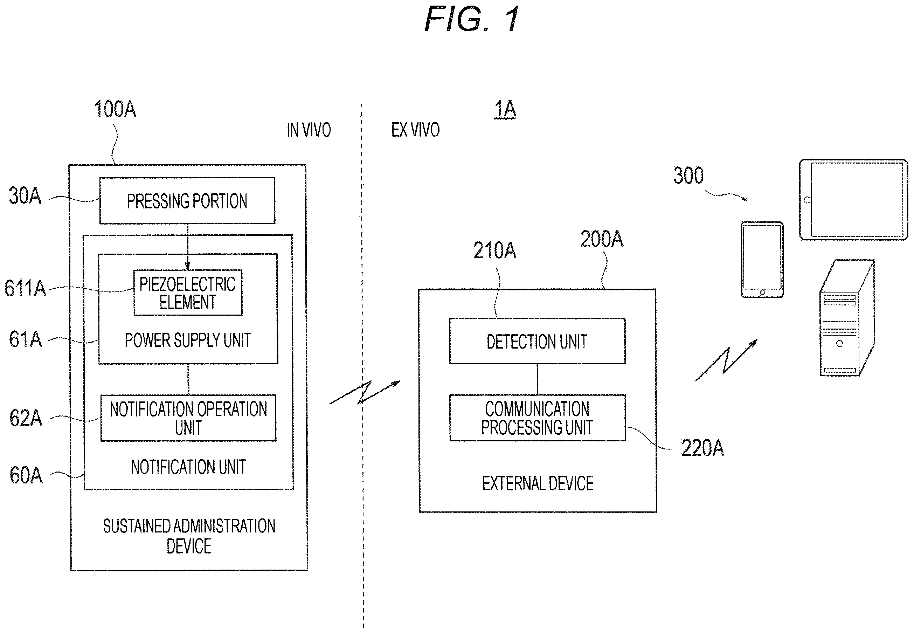

A medical system 1A according to a first embodiment will be described with appropriate reference to Figs. 1 to 3.

As illustrated in Fig. 1, the medical system 1A includes a sustained administration device 100A that is indwelled in a living body and sustainably administers (extendedly releases) a substance X to be administered such as a drug into the living body, and an external device 200A that is disposed outside the living body and is capable of detecting a notification operation emitted from the sustained administration device 100A and notifying an external communication destination 300.

The medical system 1A has a function of notifying the outside when a predetermined failure occurs in the sustained administration device 100A indwelled in the living body. Specifically, in the sustained administration device 100A indwelled in the living body, the medical system 1A performs a predetermined notification operation when the internal pressure of the inner cavity 11A exceeds a predetermined pressure along with the stop of the driving of the plug 40A, detects the notification operation by the external device 200A arranged outside the living body, and notifies the predetermined communication destination 300 of the notification operation.

### <Sustained administration device>

The sustained administration device 100A is indwelled in a body lumen (vascular, tracheal, intestinal, etc.) or in a body tissue such as a subcutaneous, muscle, bone, or organ in order to sustainably release the substance X to be administered in the living body, and can release the substance X to be administered under a predetermined administration condition (conditions related to administration such as administration period, dose, etc.). The sustained administration device 100A can extendedly release a substance over a long period of time (at least weeks to months, even years).

The substance X to be administered from the sustained administration device 100A is a fluid composition that can express a predetermined effect by extended release to the living body that is the administration target and can be extendedly released from the device. The substance X to be administered is, for example, a medical agent (liquid preparation) intended for treatment of a predetermined disease. The medical agent includes a drug. The drug can be any physiologically or pharmacologically active substance, in particular one known to be delivered to a human or animal body. The drug includes, but is not limited to, a drug acting on a peripheral nerve, an adrenergic receptor, a cholinergic receptor, a skeletal muscle, a cardiovascular system, a smooth muscle, a vascular system, a synoptic site, a nerve exchanger junction site, an endocrine and hormonal system, an immune system, a reproductive system, a skeletal system, a local hormonal system, a digestive and excretory system, a histamine system, or a central nervous system. In addition, the drug includes, but is not limited to, a drug to be used for treatment of infectious diseases, chronic pain, diabetes, autoimmune diseases, endocrine diseases, metabolic abnormalities, and rheumatic diseases. In addition, the drug includes, but is not limited to, peptides, protein, polypeptides (for example, enzyme, hormone, cytokine), nucleic acid, oligonucleotides, viruses, viral vectors, plasmids, nucleoprotein, polysaccharide, glycoprotein, lipoprotein, cells, steroid, analgesic, a local anesthetic, an antibiotic formulation, an anti-inflammatory corticosteroid, eye drops, other small molecules for pharmaceutical use, or synthetic analogs of these species, and mixtures thereof, and the like.

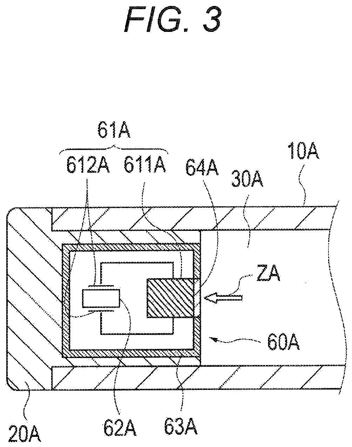

As illustrated in Fig. 2 or Fig. 3, the sustained administration device 100A generally includes a main body portion 10A, a liquid permeation portion 20A, a pressing portion 30A, a plug 40A, and a release portion 50A. In addition, the sustained administration device 100A includes a notification unit 60A that performs a predetermined notification operation to the outside when the internal pressure of the inner cavity 11A or the pressing portion 30A reaches a predetermined pressure.

The main body portion 10A is constituted with a hollow cylindrical member including the inner cavity 11A and constituting a housing of the sustained administration device 100A. The liquid permeation portion 20A, the pressing portion 30A, the plug 40A, and the release portion 50A are arranged in order from the proximal end toward the distal end of the main body portion 10A.

The main body portion 10A has a first space Y1 and a second space Y2 partitioned by the plug 40A. The first space Y1 is a space defined by the liquid permeation portion 20A and the plug 40A (a space between the attachment end of the liquid permeation portion 20A and the proximal end of the plug 40A in the main body portion 10A), and is a space in which the pressing portion 30A is accommodated. The second space Y2 is a space defined by the plug 40A and the release portion 50A (a space between the proximal end of the plug 40A and the proximal end of the release portion 50A), and is a space in which the plug 40A and the substance X to be administered are accommodated. The second space Y2 constitutes a sliding region of the plug 40A.

Spaces of the first space Y1 and the second space Y2 are enlarged or reduced as the plug 40A slides before and after the sustained administration device 100A is driven. In other words, in the first space Y1, when the sustained administration device 100A starts drive, the plug 40A slides toward the downstream side by the pressing portion 30A, whereby the space gradually expands. In the second space Y2, when the sustained administration device 100A starts drive, the plug 40A slides toward the downstream side by the pressing portion 30A, and the space is gradually reduced.

In the main body portion 10A, in order to maintain the release accuracy of the substance X to be administered by the sliding of the plug 40A, it is necessary to prevent at least the inner cavity 11A in the sliding region of the plug 40A from being crushed by buckling or the like and the shape of the inner cavity 11A (cross-sectional shape intersecting the axial direction) from being deformed to inhibit the sliding of the plug 40A. As a constituent material of the main body portion 10A, a resin material (acrylonitrile polymers, halogenated polymers, polyimide, polysulfone, polycarbonate, polyethylene, polypropylene, polyvinyl chloride-acrylic acid copolymer, polycarbonate-acrylonitrile-butadiene-styrene, polystyrene, and the like) or a metal material (stainless steel, titanium, nickel, aluminum, vanadium, platinum, tantalum, gold, and alloys thereof, and gold plated alloy iron, platinum plated alloy iron, cobalt chromium alloy, titanium nitride-coated stainless steel, and the like) which is non-invasive or minimally invasive to a living body and known in the medical field and the like can be applied.

The liquid permeation portion 20A is disposed on the proximal end side of the main body portion 10A, isolates the living body from inside of the sustained administration device 100A, and permeates only a liquid component contained in a body fluid in the living body. The liquid permeation portion 20A is constituted with a member having a solid-liquid separation function capable of permeating only a moisture in a body fluid.

As an example, a semipermeable membrane including a plasticized cellulose-based material, reinforced polymethyl methacrylates (PMMA) such as hydroxyl ethyl methacrylate (HEMA), and elastomer materials such as polyurethanes and polyamides, polyether-polyamide copolymers, thermoplastic copolyesters, and a mixture thereof can be applied as the liquid permeation portion 20A.

The pressing portion 30A is located on the downstream side with respect to the liquid permeation portion 20A in the main body portion 10A and presses the plug 40A toward the downstream side. The pressing portion 30A is an osmotic engine that swells with moisture that has permeated the liquid permeation portion 20A using the principle of osmotic pressure. The pressing portion 30A gradually swells with the moisture that has permeated the liquid permeation portion 20A and slides the plug 40A toward the downstream side by pressing action caused by increase in an internal pressure of the first space Y1 due to the swelling.

A constituent material, a size, and the like, of the pressing portion 30A can be appropriately determined so as to achieve a pressing speed (that is, a swelling speed of the pressing portion 30A) of the plug 40A according to use conditions (an extended release period, an administration amount per unit time, and the like, of the substance X to be administered). The sustained administration device 100A can adjust the period and amount of the sustained release by the configuration of the osmotic engine composing the pressing portion 30A, and the water absorption speed, water absorption rate, and the shape such as thickness of the semipermeable membrane composing the liquid permeation portion 20A, and the like.

The osmotic engine constituting the pressing portion 30A includes an osmotic agent ZA containing a hydrophilic polymer, an osmotic substance, or a mixture thereof. When the osmotic substance is a non-electrolyte, the osmotic agent ZA may be configured to further contain an electrolyte. In addition, the pressing portion 30A may have any configuration as long as it can sustainably press at least the plug 40A toward the downstream side, and may have a mechanical configuration using another driving source (motor or the like) without being limited to the osmotic engine. The solubility, dissolution rate, swelling rate, osmotic pressure of the solution, and the like of the osmotic agent ZA, or the driving speed of the mechanical driving source can be arbitrarily adjusted.

The plug 40A includes a columnar base portion 41A extending in the longitudinal direction of the main body portion 10A and an annular projecting stripe-shaped protruding portion 42A protruding on the radially outer periphery of the base portion 41A. The plug 40A is pressed by the pressing portion 30A and moves to the downstream side to push out the substance X to be administered stored in the second space Y2 toward the release portion 50A. The plug 40A is located on the downstream side with respect to the pressing portion 30A in the main body portion 10A, has an outer peripheral surface in liquid-tight contact with the inner peripheral surface of the inner cavity 11A of the main body portion 10A and is slidably disposed in the inner cavity 11A.

A plurality of the protruding portions 42A are provided on the outer surface of the base portion 41A, and the vertex portion protruding to the outermost side abuts on the inner peripheral surface of the inner cavity 11A of the main body portion 10A. The protruding portion 42A seals between the inner peripheral surface of the inner cavity 11A and the protruding portion 42A so that the substance X to be administered does not leak to the upstream side with respect to the plug 40A.

The number and positions of the protruding portion 42A are not particularly limited. In addition, the plug 40A may have a configuration in which the protruding portion 42A is not provided, that is, a configuration in which the outer peripheral surface of the base portion 41A is in direct contact with the inner peripheral surface of the inner cavity 11A to be liquid-tight. As a result, frictional force between the plug 40A and the inner peripheral surface of the main body portion 10A is adjusted, and stable sliding is enabled.

The material composing the plug 40A is a flexible material that maintains adherence (liquid tightness) with respect to the inner peripheral surface of the inner cavity 11A of the main body portion 10A. The flexible material is preferably an elastic material, and examples of the elastic material include various rubber materials (in particular, those subjected to vulcanization treatment) such as natural rubber, isoprene rubber, butyl rubber, chloroprene rubber, nitrile-butadiene rubber, styrene-butadiene rubber, and silicone rubber, styrene-based elastomers, hydrogenated styrene-based elastomers, and styrene-based elastomers mixed with polyolefins such as polyethylene, polypropylene, polybutene, and α-olefin copolymers, oils such as liquid paraffin and process oil, and powder inorganic substances such as talc, cast, and mica. Furthermore, a polyvinyl chloride-based elastomer, an olefin-based elastomer, a polyester-based elastomer, a polyamide-based elastomer, a polyurethane-based elastomer, or a mixture thereof can be used as the constituent material. As the constituent material, in particular, a diene-based rubber, a styrene-based elastomer, or the like can be applied from the viewpoint of having elastic characteristics and enabling γ-ray sterilization, electron beam sterilization, and highpressure steam sterilization. The plug 40A only requires to be slidable in the inner cavity 11A in a liquid-tight manner. Thus, the base portion 41A and the protruding portion 42A of the plug 40A may be constituted with a flexible material or an elastic material, or for example, only the protruding portion 42A abutting on the inner cavity 11A may be constituted with an elastic material, and the base portion 41A may be constituted with a hard material having no elasticity. The surface of the plug 40A may be coated with a coating having functions such as slipperiness, water repellency, and corrosion resistance.

The release portion 50A is disposed on the distal end side of the main body portion 10A, and releases the substance X to be administered pushed out by the plug 40A into the living body. The release portion 50A has a hole penetrating a columnar member having a predetermined thickness along an axial direction in order to efficiently administer the substance X to be administered into the living body. When the release portion 50A has a plurality of through holes, the substance X to be administered can be diffused into the living body without agglomerating at one place. The shape, size, and number of the through holes formed in the release portion 50A can be appropriately determined in consideration of properties of the substance X to be administered, ease of diffusion of the substance X to be administered at the place where the sustained administration device 100A is indwelled, and the like. The shape of the through hole is not limited to a linear shape, and may be, for example, a spiral shape. Further, it may penetrate to the central portion of the release portion 50A in the axial direction, and penetrate to the outside of the living body in a substantially V shape from there through the main body portion 10A in the circumferential direction.

As illustrated in Fig. 3, when the movement of the plug 40A is stopped due to clogging of the release portion 50A, sliding failure of the plug 40A, or the like, and the internal pressure of the inner cavity 11A and the pressing portion 30A exceed a predetermined threshold value A, the notification unit 60A performs a notification operation of notifying that an abnormality has occurred outside. The notification unit 60A includes a power supply unit 61A that is driven as the internal pressure of the inner cavity 11A increases, and a notification operation unit 62A that is driven by the drive voltage from the power supply unit 61A and performs a predetermined notification operation. In the notification unit 60A, in order to prevent a short circuit due to entry of liquid or the like, the housing 63A serving as a main body is made of a liquid-impermeable member and disposed in the liquid permeation portion 20A, and a contact portion with the pressing portion 30A is made of a liquid-impermeable membrane member 64A capable of transmitting an increase in internal pressure of the inner cavity 11A to the power supply unit 61A, specifically, a piezoelectric body 611A to be described later. As described above, the liquid passage portion 60A is sealed by the housing 63A and the membrane member 64A, and is configured to transmit the pressure rise of the pressing portion 30A due to the increase in the internal pressure of the inner cavity 11A to the piezoelectric body 611A via the membrane member 64A as the internal pressure of the inner cavity 11A rises.

The power supply unit 61A includes a piezoelectric body 611A made of a piezoelectric ceramic material and a pair of electrodes 612A, and includes a known piezoelectric element that generates a drive voltage by an internal pressure of the pressing portion 30A resulting from an increase in the internal pressure when the internal pressure of the inner cavity 11A exceeds a threshold value A. In the power supply unit 61A, when the movement of the plug 40A stops due to some failure and the internal pressure of the inner cavity 11A exceeds the threshold value A, this internal pressure fluctuation is applied to the piezoelectric body 611A, and a voltage is generated by the piezoelectric effect. The power supply unit 61A supplies (applies) the generated voltage to the notification operation unit 62A as a drive voltage.

The "threshold value A" can be set to a pressure value that is at least higher than the internal pressure of the inner cavity 11A when the plug 40A is normally driven and lower than the pressure at which a burst of the substance X to be administered can occur due to the increase in the internal pressure resulting from the stop of the plug 40A.

The notification operation unit 62A performs a predetermined notification operation and outputs notification information to the outside. The notification operation unit 62A includes a device that can perform a predetermined notification operation by being driven by a drive voltage applied from the power supply unit 61A.

The "notification operation" is an operation of notifying the outside (outside the living body) that the driving of the plug 40A has stopped and the pressure in the inner cavity 11A has exceeded the threshold value A, that is, an abnormality occurred in the sustained administration device 100A. The notification operation unit 62A outputs the physical event generated by the notification operation to the outside as notification information. The notification operation unit 62A can include a vibrator that is driven by a drive voltage applied from the power supply unit 61A to generate vibration at a predetermined cycle. The notification operation corresponds to, for example, an operation of generating a physical event such as heat, magnetic field, sound, light, radio wave, or sound wave in addition to vibration.

As described above, when the movement of the plug 40A is stopped due to some failure, the internal pressure of the inner cavity 11A increases with the stop of movement of the plug, and the internal pressure exceeds the threshold value A, the power supply unit 61A operates to generate the drive voltage, and this drive voltage is applied to the notification operation unit 62A, so that the notification unit 60A performs the notification operation to notify the outside. The notification subject (for example, a medical worker such as a doctor) can learn that an abnormality has occurred in the sustained administration device 100A via the external device 200A.

### <External Device>

As illustrated in Fig. 1, the external device 200A is disposed outside the living body. The external device 200A detects notification information from the notification unit 60A of the sustained administration device 100A, and notifies the communication destination 300 that an abnormality has occurred in the sustained administration device 100A. The external device 200A includes a detection unit 210A that detects the notification operation, and a communication processing unit 220A that notifies the communication destination 300 that the detection unit 210A has detected the notification operation.

The "communication destination" is a device possessed or managed by a notification subject such as a manager who manages the sustained administration device 100A or a patient in which the sustained administration device 100A is indwelled. Examples of the device of the communication destination 300 include a mobile terminal (smartphone, tablet terminal, mobile phone, etc.), an external terminal such as a PC (including an external management server and the like), and a notification device having a predetermined notification function (a display device such as a sound output device having a sound generation function such as a buzzer sound or a sound, an alarm lamp having a lighting/blinking function or a character display function, or a display device). However, the device serving as the communication destination 300 is not limited to the exemplified device, and may be any device as long as it can notify the notification subject of the abnormality of the sustained administration device 100A.

The detection unit 210A includes a device capable of detecting notification information generated by the notification operation of the notification unit 60A. The detection unit 210A detects notification information from the notification unit 60A, and outputs a detection result to the communication processing unit 220A. In a case where the notification information of the notification unit 60A is "vibration", the detection unit 210A can include a vibration sensor capable of detecting displacement of the living body surface derived from this vibration.

The communication processing unit 220A generates an abnormality notification signal based on the detection result from the detection unit 210A and transmits the abnormality notification signal to the communication destination 300. The communication processing unit 220A can include a network interface that controls wireless communication with other devices via a communication network according to a predetermined protocol. The communication processing unit 220A only needs to have a function capable of communicating with the communication destination 300, such as short-range wireless communication with a communication distance of about several centimeters to several tens of meters, such as Bluetooth (registered trademark) or Wi-Fi, or wireless communication using a carrier line network provided by a communication company.

Note that, in a case where the external device 200A has a function capable of notifying the notification subject such as the sound output device or the display device of the abnormality of the sustained administration device 100A without performing the communication processing, the communication processing unit 220A can be omitted. In this case, if the external device 200A is configured to detect the notification operation of the notification unit 60A by the detection unit 210A and perform a predetermined notification operation (sound, light output, character display, etc.) based on the detection result of the detection unit 210A, it is possible to notify the notification subject of the abnormality of the sustained administration device 100A.

In addition, the medical system 1A is configured to detect the notification information from the sustained administration device 100A by the external device 200A and confirm the presence or absence of abnormality of the sustained administration device 100A. However, when the notification information from the sustained administration device 100A is an event that can be confirmed by the notification subject, the abnormality of the sustained administration device 100A can be confirmed without using the external device 200A. For example, in a case where the notification information of the sustained administration device 100A is "sound", the notification subject can know the abnormality of the sustained administration device 100A by listening to a specific sound (communication sound) serving as the notification information.

In the medical system 1A according to the first embodiment described above, the sustained administration device 100A is appropriately introduced into the living body by a percutaneous or surgical method or the like after being filled with the substance X to be administered for the administration target. In addition, the external device 200A is disposed at a position where the notification operation emitted from the sustained administration device 100A in vitro is detectable, for example, on the surface of the living body in the vicinity of the indwelling position of the sustained administration device 100A.

It is assumed that the sustained administration device 100A stops the movement of the plug 40A due to some failure. At that time, in the notification unit 60A, when the internal pressure of the inner cavity 11A exceeds the threshold value A due to the stop of movement of the plug 40A, a pressure exceeding the threshold value A is applied to the power supply unit 61A, whereby a drive voltage is generated. The notification unit 60A drives the notification operation unit 62A by the drive voltage generated by the power supply unit 61A to perform a predetermined notification operation.

When the notification information is output by the notification operation of the notification unit 60A, the external device 200A detects the notification information by the detection unit 210A, and transmits an abnormality notification signal to the communication destination 300 (for example, a mobile terminal possessed by the notification subject) via the communication processing unit 220A. As a result, the notification subject can immediately confirm the abnormality of the sustained administration device 100A. Therefore, when a failure occurs in the sustained administration device 100A, even if the notification subject is at a place away from the patient in which the sustained administration device 100A is indwelled, it is possible to promptly respond.

### [Second Embodiment]

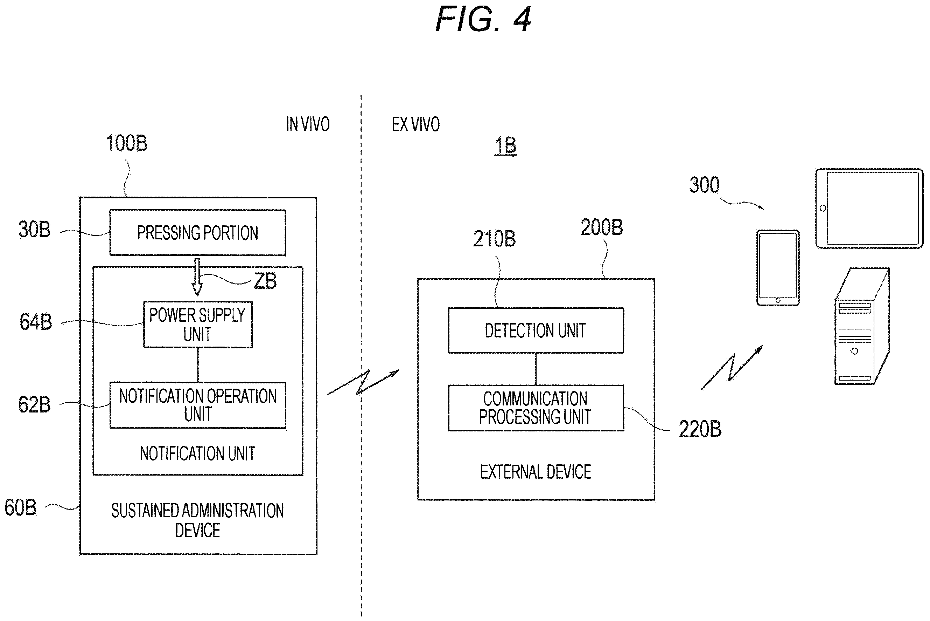

Next, a medical system 1B according to a second embodiment will be described with appropriate reference to Figs. 4 to 6.

Note that, in the medical system 1B according to the second embodiment, constituent elements having functions equivalent to those of the first embodiment described above are denoted by the same or related reference numerals, detailed description thereof is omitted with no particular reference. The configuration, the members, the method of use, and the like may be similar to those of the first embodiment described above.

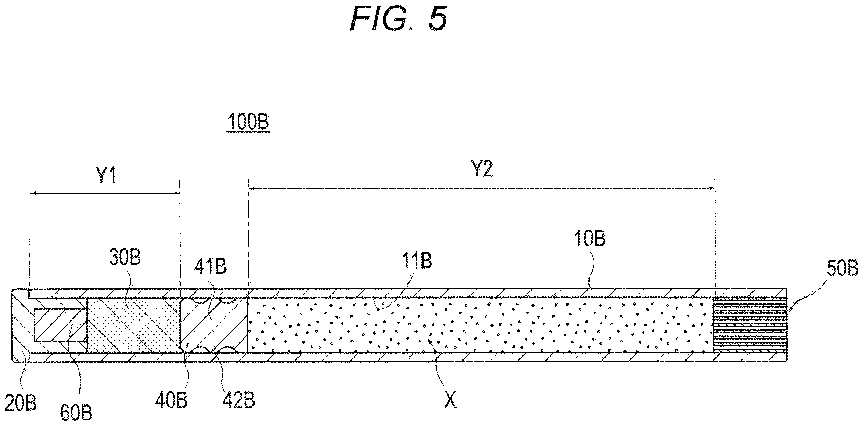

As illustrated in Figs. 4 and 5, the medical system 1B is configured with a sustained administration device 100B and an external device 200B. The sustained administration device 100B generally includes a main body portion 10B, a liquid permeation portion 20B, a pressing portion 30B, a plug 40B, a release portion 50B, and a notification unit 60B.

As illustrated in Figs. 4 and 5, in the medical system 1B according to the second embodiment, the configuration of the notification unit 60B is mainly different from the configuration of the notification unit 60A of the first embodiment. In addition, the pressing portion 30B is configured with an osmotic engine using an osmotic agent ZB containing an electrolyte as a conductor necessary for driving the notification unit 60B. When the plug 40B stops moving due to an increase in the internal pressure of the inner cavity 11B, the plug 40B inhibits the expansion of the first space Y1 to the downstream side, and the osmotic agent ZB flows out to the upstream side and flows into the notification unit 60B.

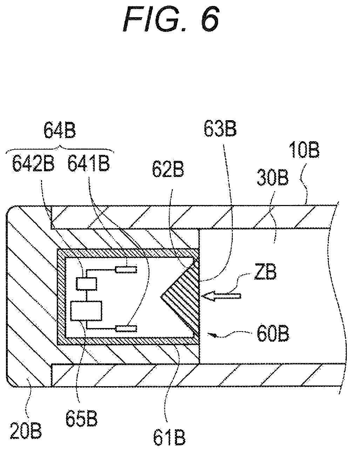

As illustrated in Fig. 5 or 6, the notification unit 60B is configured to include: a housing 61B serving as a main body; a liquid passage port 62B of the osmotic agent ZB that is provided in the housing 61B and flows in from the pressing portion 30B;a blocking portion 63B that, in a normal time, blocks the liquid passage port 62B and, when the internal pressure of the inner cavity 11B exceeds a predetermined threshold value B, opens the liquid passage port 62B by the pressure of the pressing portion 30B resulting from the increase in the internal pressure; a power supply unit 64B that can be energized by the osmotic agent ZB flowing in from the liquid passage port 62B and generates a drive voltage; and a notification operation unit 65B that performs a predetermined notification operation when the drive voltage is applied from the power supply unit 64B. The notification unit 60B is disposed on the distal end side of the liquid permeation portion 20B so that a part of the osmotic agent ZB from the pressing portion 30B can flow in.

Similarly to the threshold value A, the "threshold value B" can be set to a pressure value that is at least higher than the internal pressure of the inner cavity 11B when the plug 40B is normally driven and lower than the pressure at which a burst of the substance X to be administered can occur due to the increase in the internal pressure resulting from the stop of movement of the plug 40B.

The housing 61B is configured with a material having liquid impermeability in order to prevent a short circuit of the power supply unit 64B and the notification operation unit 65B. The housing 61B is disposed on the distal end side of the liquid permeation portion 20B so that the osmotic agent ZB can flow in. The liquid passage portion 60B is sealed by the housing 61B and a blocking portion 63B provided in the liquid passage port 62B.

The liquid passage port 62B is formed on the distal end side of the housing 61B facing the pressing portion 30B so that the osmotic agent ZB can flow in. The liquid passage port 62B is closed by the blocking portion 63B in a normal time, and when the internal pressure of the inner cavity 11B reaches a pressure exceeding the threshold value B, that is, when an abnormality occurs in the sustained administration device 100B, the blocking by the blocking portion 63B is released. As a result, the liquid passage port 62B is opened to allow the osmotic agent ZB to flow in.

The blocking portion 63B is formed of a member capable of releasing the closed state of the liquid passage port 62B when the internal pressure of the inner cavity 11B exceeds the threshold value B, and closes the liquid passage port 62B at the time of normal driving so that moisture or the like having permeated the osmotic agent ZB or the liquid permeation portion 20B does not flow into the housing 61B. The blocking portion 63B releases the blockage of the liquid passage port 62B when the internal pressure of the inner cavity 11B becomes a pressure exceeding the threshold value B due to the occurrence of the abnormality.

The blocking portion 63B can be configured with a check valve, a membrane member, or the like having a function that the liquid passage port 62B can be opened when the internal pressure of the inner cavity 11B exceeds the threshold value B. In a case where the blocking portion 63B is configured by a check valve, the valve body, which blocks the liquid passage port 62B by the pressure of when the internal pressure of the inner cavity 11B exceeds the threshold value B, separates (or opens) from the liquid passage port 62B to open the liquid passage port 62B. In addition, in a case where the blocking portion 63B is formed of a membrane member, when the internal pressure of the inner cavity 11B exceeds the threshold value B, at least a part of the membrane member is broken by the pressing force of the osmotic agent ZB to open the liquid passage port 62B.

The power supply unit 64B generates and supplies a drive voltage necessary for driving the notification operation unit 65B. The power supply unit 64B is configured with a battery 642B to which a pair of electrodes 641B arranged apart from each other to secure an insulation distance in the housing 61B is connected. In the power supply unit 64B, the electrolyte contained in the osmotic agent ZB is used as a conductor, and the electrode 641B is immersed in the osmotic agent ZB having flowed in through the liquid passage port 62B, whereby electricity can be supplied. Therefore, the power supply unit 64B is not driven unless the internal pressure of the inner cavity 11B reaches a pressure exceeding the threshold value B and the blockage of the liquid passage port 62B by the blocking portion 63B is released. The power supply unit 64B supplies (applies) a drive voltage generated by conduction between the osmotic agent ZB having flowed in through the liquid passage port 62B and the electrode 641B to the notification operation unit 65B. The type and the like of the battery configuring the power supply unit 64B are not particularly limited as long as the battery can be accommodated in the housing 61B and can generate a drive voltage that can drive the notification operation unit 65B.

The notification operation unit 65B is driven by the drive voltage from the power supply unit 64B to perform a predetermined notification operation. As with the notification operation unit 62A of the first embodiment, the notification operation outputs the physical event generated by the notification operation to the outside as notification information.

In addition, the medical system 1B includes an external device 200B including a detection unit 210B and a communication processing unit 220B, detects a notification operation of the notification unit 60B, and notifies the communication destination 300 that an abnormality has occurred in the sustained administration device 100B.

In the medical system 1B according to the second embodiment described above, the sustained administration device 100B is indwelled in the living body in a state of being filled with the substance X to be administered for the administration target, and the external device 200B is disposed at a position where the notification operation emitted from the sustained administration device 100B outside the living body can be detected.

It is assumed that the sustained administration device 100B stops the movement of the plug 40B due to some failure. In this case, when the internal pressure of the inner cavity 11B exceeds the threshold value B due to stop of movement of the plug 40B, the notification unit 60B causes the pressure exceeding the threshold value B to act on the blocking portion 63B to open the liquid passage port 62B. In the power supply unit 64B, the electrode 641B is conducted using the osmotic agent ZB flowing into the housing 61B from the opened liquid passage port 62B as a conductor, and generates a drive voltage. The notification unit 60B drives the notification operation unit 65B by the drive voltage generated by the power supply unit 64B to perform a predetermined notification operation.

When the notification information is output by the notification operation of the notification unit 60B the external device 200B detects the notification information by the detection unit 210B, and transmits an abnormality notification signal to the communication destination 300 (for example, a mobile terminal possessed by the notification subject) via the communication processing unit 220B. As a result, the notification subject can immediately confirm the abnormality of the sustained administration device 100B. Therefore, when a failure occurs in the sustained administration device 100B, even if the notification subject is at a place away from the patient in which the sustained administration device 100B is indwelled, it is possible to promptly respond.

### [Third Embodiment)

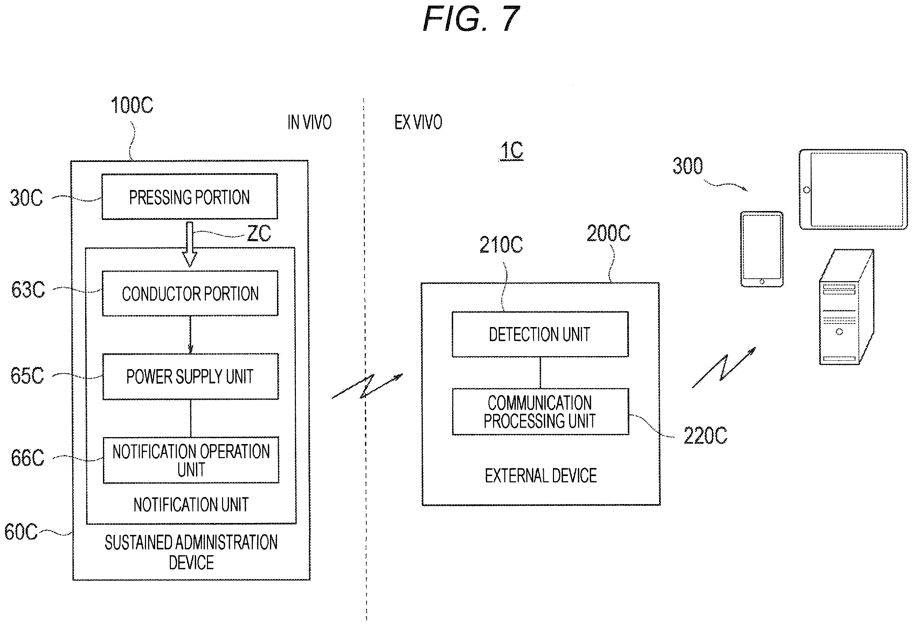

Next, a medical system 1C according to a third embodiment will be described with appropriate reference to Figs. 7 to 9.

Note that, as for the medical system 1C according to the third embodiment, constituent elements having the similar functions as those in the above-described first and second embodiments may be denoted by the same reference numerals and detailed description thereof may be omitted, and configurations, members, usage methods, and the like, that are not particularly mentioned may be similar to those in the above-described first and second embodiments.

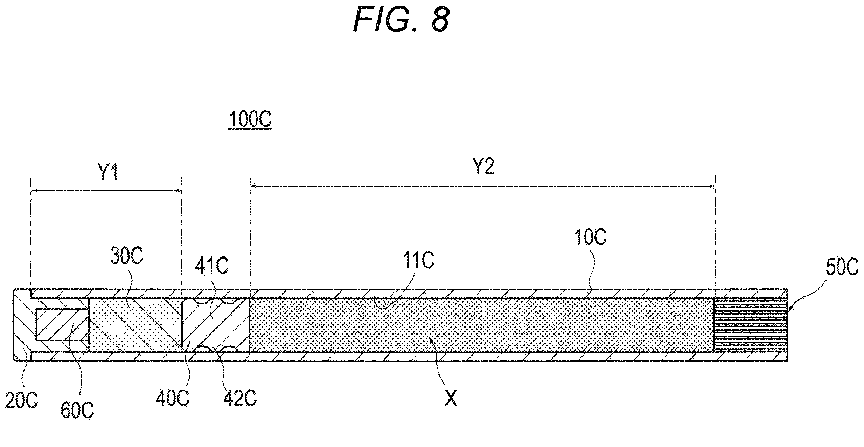

As illustrated in Figs. 7 and 8, the medical system 1C is configured with a sustained administration device 100C and an external device 200C. The sustained administration device 100C generally includes a main body portion 10C, a liquid permeation portion 20C, a pressing portion 30C, a plug 40C, a release portion 50C, and a notification unit 60C.

As illustrated in Figs. 7 and 8, in the medical system 1C according to the third embodiment, the configuration of the notification unit 60C is mainly different from the configuration of the notification unit 60A of the first embodiment and the notification unit 60B of the second embodiment. In addition, the pressing portion 30C is configured by an osmotic engine in order to perform energization using a pressing action by inflow of the osmotic agent ZC as a drive source of the notification unit 60C. When the internal pressure of the inner cavity 11C increases due to stop of movement of the plug 40C, the plug 40C inhibits the expansion to the downstream side, and the osmotic agent ZC flows out to the upstream side and flows into the notification unit 60C.

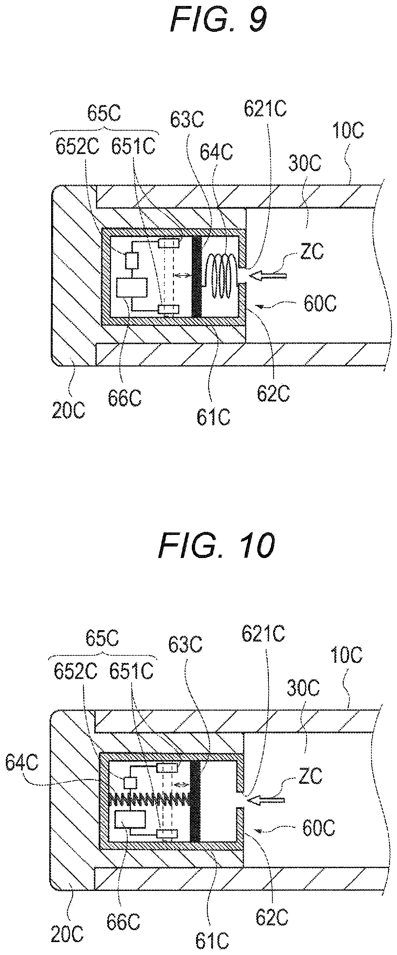

As illustrated in Fig. 8 or 9, the notification unit 60C includes: a housing 61 C serving as a main body; a liquid passage portion 62C provided in the housing 61C and provided with a liquid passage port 621C of the osmotic agent ZC flowing in from the pressing portion 30C;a conductor portion 63C that moves by the osmotic agent ZC flowing in from the liquid passage portion 62C when the internal pressure of the inner cavity 11C exceeds a predetermined threshold value C; a holding portion 64C that holds the conductor portion 63C on the liquid passage portion 62C side with a force less than the pressing force of the osmotic agent ZC; a power supply unit 65C that generates a drive voltage by conduction of the electrode 651C by the conductor portion 63C thus moved; and a notification operation unit 66C that performs a predetermined notification operation when a drive voltage is applied from the power supply unit 65C. The notification unit 60C is disposed on the distal end side of the liquid permeation portion 20C so that the pressing force by the osmotic agent ZC from the pressing portion 30C acts.

Similarly to the threshold values A and B, the "threshold value C" can be set to a pressure value that is at least higher than the internal pressure of the inner cavity 11C when the plug 40C is normally driven and lower than the pressure at which a burst of the substance X to be administered can occur due to the increase in the internal pressure resulting from the stop of movement of the plug 40C.

The housing 61C is configured with a material having liquid impermeability in order to prevent a short circuit of the power supply unit 65C and the notification operation unit 66C. The housing 61C is disposed on the distal end side of the liquid permeation portion 20C so that the osmotic agent ZC can flow in.

The liquid passage portion 62C is formed with a liquid passage port 621C through which the osmotic agent ZC can pass, and is formed on the distal end side of the housing 61C facing the pressing portion 30C so that the osmotic agent ZC can flow in. When the internal pressure of the inner cavity 11B reaches a pressure exceeding the threshold value C, that is, when an abnormality occurs in the sustained administration device 100B, the pressure by the osmotic agent ZC flowing in from the liquid passage portion 62C increases, and the conductor portion 63C is driven to the upstream side.

The liquid passage port 621C of the liquid passage portion 62C is always open, and in addition to the osmotic agent ZC, moisture having permeated the liquid permeation portion 20C can also flow in. However, since the osmotic agent ZC is prevented from entering the upstream side from the conductor portion 63C by the conductor portion 63C disposed on the distal end side of the power supply unit 65C and the notification operation unit 66C, the space on the upstream side of the conductor portion 63C in the housing 61C is sealed, and the power supply unit 65C and the notification operation unit 66C are not shortcircuited.

The conductor portion 63C is configured with a conductive material having electrical conductivity or a material containing a conductive material. The conductor portion 63C functions as a conductor for conducting the electrode 651C of the power supply unit 65C. The conductor portion 63C can be configured with, for example, a conductive metal plate.

The conductor portion 63C is held movably along the axial direction of the main body portion 10C by the holding portion 64C. When the internal pressure of the inner cavity 11C exceeds the threshold value C, the conductor portion 63C moves against the tensile force of the holding portion 64C when pressed toward the upstream side by the osmotic agent ZC flowing from the liquid passage portion 62C. The conductor portion 63C is pushed and moved from the initial position to the conduction position (the contact position with the electrode 651C) by the pressing action of the inflow osmotic agent ZC, and a part (both end portions) thereof comes into contact with the electrode 651C. As a result, the power supply unit 65C becomes electromotive.

The holding portion 64C holds the conductor portion 63C. The force for holding the conductor portion 63C of the holding portion 64C pulls and holds the conductor portion 63C toward the liquid passage portion 62C (downstream side) with a force (tensile force) weaker than the pressing force by the osmotic agent ZC flowing in from the liquid passage port 621C of the liquid passage portion 62. In other words, the tensile force of the holding portion 64C is set to a force capable of moving the conductor portion 63C from the initial position to the conduction position by the pressing force of the osmotic agent ZC flowing in from the liquid passage port 621C when the internal pressure of the inner cavity 11C exceeds the threshold value C. One end of the holding portion 64C is connected to the liquid passage portion 62C, and the other end is connected to the conductor portion 63C. The holding portion 64C can be formed of an elastic member (such as a torsion coil spring) capable of holding the conductor portion 63C with a predetermined tensile force. In addition, as illustrated in Fig. 10, the holding portion 64C may be configured with a compression spring that can expand and contract along the axial direction of the main body portion 10C, may be disposed in the internal space of the housing 61C so as to hold the conductor portion 63C, and may have a structure of resisting pressure by the osmotic agent ZC. At this time, the force of the holding portion 64C holding the conductor portion 63C is set to a force capable of moving the conductor portion 63C from the initial position to the conduction position by the pressing force of the osmotic agent ZC flowing in from the liquid passage port 621C when the internal pressure of the inner cavity 11C exceeds the threshold value C.

The power supply unit 65C generates and supplies a drive voltage necessary for driving the notification operation unit 66C. The power supply unit 65C is configured with a battery 652C to which a pair of electrodes 651C arranged apart from each other to secure an insulation distance in the housing 61C is connected. In the power supply unit 65C, the conductor portion 63C moved by the inflow of the osmotic agent ZC from the liquid passage portion 62C comes into contact with the electrode 651C, so that the electrode 651C is conducted and energized. The power supply unit 65C supplies (applies) a drive voltage generated by conduction between the conductor portion 63C and the electrode 651C to the notification operation unit 66C. The type and the like of the battery configuring the power supply unit 65C are not particularly limited as long as the battery can be accommodated in the housing 61C and can generate a drive voltage that can drive the notification operation unit 66C.

The notification operation unit 66C is driven by the drive voltage from the power supply unit 65C to perform a predetermined notification operation. As with the notification operation unit 62A of the first embodiment and the notification operation unit 65B of the second embodiment, the notification operation outputs the physical event generated by the notification operation to the outside as notification information.

In addition, the medical system 1C includes an external device 200C including a detection unit 210C and a communication processing unit 220C, detects a notification operation of the notification unit 60C, and notifies the communication destination 300 that an abnormality has occurred in the sustained administration device 100C.

In the medical system 1C according to the third embodiment described above, the sustained administration device 100C is indwelled in the living body in a state of being filled with the substance X to be administered for the administration target, and the external device 200C is disposed at a position where the notification operation emitted from the sustained administration device 100C outside the living body can be detected.

It is assumed that the sustained administration device 100C stops the movement of the plug 40C due to some failure. In this case, in the notification unit 60C, when the internal pressure of the inner cavity 11C exceeds the threshold value C due to the stop of movement of the plug 40C, the conductor portion 63C is pressed by the osmotic agent ZC flowing in from the liquid passage portion 62C and moves to the upstream side. The power supply unit 65C generates a drive voltage by conduction between the conductor portion 63C moved to the upstream side and the electrode 651C. The notification unit 60C drives the notification operation unit 66C by the drive voltage generated by the power supply unit 65C to perform a predetermined notification operation.

When the notification information is output by the notification operation of the notification unit 60C, the external device 200C detects the notification information by the detection unit 210C, and transmits an abnormality notification signal to the communication destination 300 (for example, a mobile terminal possessed by the notification subject) via the communication processing unit 220C. As a result, the notification subject can immediately confirm the abnormality of the sustained administration device 100C. Therefore, when a failure occurs in the sustained administration device 100C, even if the notification subject is at a place away from the patient in which the sustained administration device 100C is indwelled, it is possible to promptly respond.

### [Operational Effects]

As described above, the sustained administration devices 100A to 100C according to the present embodiment respectively include: the main body portions 10A to 10C respectively having the inner cavities 11A to 11C;the plugs 40A to 40C that are slidable in a liquid-tight manner in the inner cavities 11A to 11C; the pressing portions 30A to 30C that are disposed on the upstream side of the plugs 40A to 40C in the inner cavities 11A to 11C and press the plugs 40A to 40C toward the downstream side; the substance X to be administered that is accommodated on the downstream side of the plugs 40A to 40C in the inner cavities 11A to 11C and is released into a living body by sliding of the plugs 40A to 40C toward the downstream side; and the release portions 50A to 50C that are disposed on the distal end side of the main body portions 10A to 10C, and releases the substance X to be administered pushed out by the plugs 40A to 40C into the living body, the devices 100A to 100C being indwelled in a living body for sustainably extendedly releasing the substance X to be administered, and including: the notification units 60A to 60C that perform a notification operation for notifying abnormality to the outside when an internal pressure of the inner cavities 11A to 11C exceeds a predetermined threshold value due to stop of movement of the plugs 40A to 40C.

As a result, when some failure occurs in the sustained administration device 100 indwelled in the living body, the movement of the plugs 40A to 40C is stopped, and the internal pressure of the inner cavities 11A to 11C exceeds the predetermined threshold value, the occurrence of abnormality can be notified to the outside. Therefore, the notification subject can immediately know that an abnormality has occurred in the sustained administration device 100. In addition, it is possible to avoid the risk of loss of the administration effect of the substance X to be administered due to stop of extended release and the serious health hazard due to the burst of the substance X to be administered. Therefore, even a drug that causes serious side effects when excessively administered can be used as the substance X to be administered without concern, and the range of treatment options can be extended.

In the sustained administration device 100A according to the present embodiment, the notification unit 60A may be configured to include: the power supply unit 61A including the piezoelectric body 611A and the piezoelectric element that has the pair of electrodes 612A, and generates a drive voltage by a pressure applied when an internal pressure of the inner cavity 11A exceeds the threshold value A; and the notification operation unit 62A that performs a predetermined notification operation when a drive voltage is applied from the power supply unit 61A.

With such a configuration, the sustained administration device 100A can drive the notification operation unit 62A by electromotive force of the power supply unit 61A when the internal pressure of the inner cavity 11A exceeds the threshold value A, and thus can immediately notify the notification subject that the abnormality has occurred in the sustained administration device 100A.

Further, the sustained administration device 100B according to the present embodiment includes the liquid permeation portion 20B disposed on a proximal end side of the main body portion 10B, in which the pressing portion 30B includes an osmotic engine including the osmotic agent ZB that contains an electrolyte and is swollen by a liquid that permeates the liquid permeation portion 20B, and the notification unit 60B may be configured to include the liquid passage port 62B of the osmotic agent ZB, the blocking portion 63B that blocks the liquid passage port 62B during normal time and opens the liquid passage port 62B when the internal pressure of the inner cavity 11B exceeds the threshold value B, the power supply unit 64B that has the pair of electrodes 641B and generates a drive voltage by conducting the electrodes 641B with the osmotic agent ZB flowing in from the liquid passage port 62B, and the notification operation unit 65B that performs a notification operation when the drive voltage is applied from the power supply unit 64B.

With such a configuration, the sustained administration device 100B can drive the notification operation unit 65B by the power supply unit 64B being energized by the osmotic agent ZB flowing in from the liquid passage port 62B when the internal pressure of the inner cavity 11B exceeds the threshold value B, and thus can immediately notify the notification subject that the abnormality has occurred in the sustained administration device 100B.

Further, the sustained administration device 100C according to the present embodiment includes the liquid permeation portion 20C disposed on the proximal end side of the main body portion 10C, in which the pressing portion 30C includes the osmotic engine including the osmotic agent ZC that is swollen by a liquid that permeates the liquid permeation portion 20C, and the notification unit 60C may be configured to include the liquid passage portion 62C with the liquid passage port 621C for the osmotic agent ZC being formed, the conductor portion 63C that moves to the upstream side by being pushed by the osmotic agent ZC flowing in from the liquid passage portion 62C when the internal pressure of the inner cavity 11C exceeds a predetermined threshold value C, the holding portion 64C that holds the conductor portion 63C on the liquid passage portion 62C side with a force less than the pressing force of the osmotic agent ZC, the power supply unit 65C that generates a drive voltage by conduction between the conductor portion 63C that have moved and the electrode 651C, and the notification operation unit 66C that performs a notification operation by being driven by a drive voltage applied from the power supply unit 65C.

With such a configuration, the sustained administration device 100C can drive the notification operation unit 62A by conduction between the power supply unit 65C and the conductor portion 63C when the internal pressure of the inner cavity 11C exceeds the threshold value C, and thus can immediately notify the notification subject that the abnormality has occurred in the sustained administration device 100C.

The medical systems 1A to 1C according to the present embodiment include the sustained administration devices 100A to 100C having any of the configurations described above, and the external devices 200A to 200C including the detection units 210A to 210C that detect the notification operations of the notification units 60A to 60C and disposed outside the living body.

Since the notification information notified from the sustained administration devices 100A to 100C indwelled in the living body can be detected by the external devices 200A to 200C disposed outside the living body, the notification subject can immediately know that an abnormality has occurred in the sustained administration devices 100A to 100C.

Furthermore, in the medical systems 1A to 1C according to the present embodiment, the external devices 200A to 200C may include communication processing units 220A to 220C that generate a notification signal indicating that the internal pressures of the inner cavities 11A to 11C have exceeded a predetermined threshold value and transmit the notification signal to the communication destination 300.

As a result, it is possible to notify the communication destination 300 such as the mobile terminal possessed by the notification subject that the abnormality has occurred in the sustained administration device 100, so that the notification subject can immediately know that the abnormality has occurred in the sustained administration devices 100A to 100C. Therefore, when a failure occurs in the sustained administration devices 100A to 100C, even if the notification subject is at a place away from the patient in which the sustained administration devices 100A to 100C is indwelled, it is possible to promptly respond.

The present application is based on Japanese Patent Application No. 2022-049284 filed on March 25, 2022, the disclosure content of which is incorporated herein by reference in its entirety.

### Reference Signs List

1A, 1B, 1C Medical system
10A, 10B, 10C Main body portion
11A, 11B, 11C Inner cavity
20A, 20B, 20C Liquid permeation portion
30A, 30B, 30C Pressing portion
40A, 40B, 40C Plug
50A, 50B, 50C Release portion
60A, 60B, 60C Notification unit
61A, 64B, 65C Power supply unit
62A, 65B, 66B Notification operation unit
62B Liquid passage port
62C Liquid passage portion (621C Liquid passage port)
63B Blocking portion
63C Conductor portion
64C Holding portion
100A, 100B, 100C Sustained administration device
200A, 200B, 200C External Device
210A, 210B, 210C Detection unit
220A, 220B, 220C Communication processing unit
611A Piezoelectric body
612A Electrode
641B, 651C Electrode
X Substance to be administered
Y1 First space
Y2 Second space
ZA, ZB, ZC Osmotic agent

## Claims

1. A sustained administration device comprising:
a main body portion including an inner cavity;
a plug that is slidable in a liquid-tight manner in the inner cavity;
a pressing portion that is disposed on an upstream side of the plug in the inner cavity and presses the plug toward a downstream side;
a substance to be administered that is accommodated on the downstream side of the plug in the inner cavity and is released into a living body by sliding of the plug toward the downstream side; and
a release portion that is disposed on a distal end side of the main body portion and releases the substance to be administered pushed by the plug into the living body,
the sustained administration device being indwelled in the living body to sustainably extendedly release the substance to be administered,
wherein the sustained administration device comprises a notification unit that performs a notification operation for notifying abnormality to the outside when an internal pressure of the inner cavity exceeds a predetermined threshold value due to stop of movement of the plug.

2. The sustained administration device according to claim 1, wherein the notification unit comprises:
a power supply unit including a piezoelectric body and a piezoelectric element that has a pair of electrodes, and generates a drive voltage by a pressure applied when an internal pressure of the inner cavity exceeds the threshold value; and
a notification operation unit that performs the notification operation when the drive voltage is applied from the power supply unit.

3. The sustained administration device according to claim 1, further comprising a liquid permeation portion disposed on a proximal end side of the main body portion, wherein
the pressing portion includes an osmotic engine including an osmotic agent that contains an electrolyte and is swollen by a liquid that permeates the liquid permeation portion, and
the notification unit is configured to include
a liquid passage port for the osmotic agent,
a blocking portion that blocks the liquid passage port during normal time and opens the liquid passage port when the internal pressure of the inner cavity exceeds the threshold value,
a power supply unit that has a pair of electrodes and generates a drive voltage by conducting the electrodes with the osmotic agent flowing in from the liquid passage port, and
a notification operation unit that performs the notification operation when the drive voltage is applied from the power supply unit.

4. The sustained administration device according to claim 1, further comprising a liquid permeation portion disposed on a proximal end side of the main body portion, wherein
the pressing portion includes an osmotic engine including an osmotic agent that is swollen by a liquid that permeates the liquid permeation portion, and
the notification unit is configured to include
a liquid passage portion with a liquid passage port for the osmotic agent being formed,
a conductor portion that is pushed by the osmotic agent flowing in from the liquid passage portion to move to a proximal end side when the internal pressure of the inner cavity exceeds the threshold value,
a holding portion that holds the conductor portion on the liquid passage portion side with a force less than a pressing force due to the osmotic agent flowing in,
a power supply unit that generates a drive voltage by conduction between the conductor portion that have moved and the electrode, and
a notification operation unit that is driven by the drive voltage applied from the power supply unit to perform the notification operation.

5. A medical system comprising:
the sustained administration device according to any one of claims 1 to 4; and
an external device that is disposed outside the living body and includes a detection unit that detects the notification operation of the notification unit.

6. The medical system according to claim 5, wherein the external device includes a communication processing unit that generates a notification signal indicating that the internal pressure of the inner cavity exceeds the threshold value and transmits the notification signal to a communication destination.
